# EUROPEAN PATENT APPLICATION

(11) **EP 2 246 437 A1**
(43) Date of publication of application: **03.11.2010**
(21) Application number: 09158971.3
(22) Date of filing: 28.04.2009
(51) Int. Cl.: C12P 7/10

(54) **Fermentation**

(71) Applicant: Sekab E-Technology AB, 891 26 Örnsköldsvik (SE)
(72) Inventor: Grundberg, Hans, SE-893 91, BJÄSTA (SE)
(74) Representative: Henriksson, Dan Ragnar Mikael

(57) **Abstract**

The present invention provides a method for providing a target chemical, comprising: a) providing a cellulose-derived slurry comprising fermentable saccharides in a container; b) separating a liquid fraction comprising part of said fermentable saccharides from said cellulose-derived slurry; c) transferring said liquid fraction to a fermentor; d) fermenting fermentable saccharides in said liquid fraction in said fermentor to provide a fermented liquid comprising said target chemical; and e) returning said fermented liquid to said container. Further, the present invention provides a system comprising: a container for containing a cellulose-derived slurry comprising fermentable saccharides; a separator for separating a liquid fraction comprising fermentable saccharides from said slurry, which separator is connected to said container; a fermentor for fermenting at least part of said fermentable saccharides in said liquid fraction to yield a fermented liquid, which fermentor comprises an inlet and an outlet; a first connection connecting said separator and said fermentor inlet and a second connection connecting said fermentor outlet and said container.

## Description

### Technical Field

The present invention relates to a method for preparing a target chemical from a cellulose-derived slurry, to a system for preparing a target chemical from a cellulose-derived slurry and to uses of a fermented, cellulose-derived hydrolysate.

### Background

Global warming, petroleum depletion and energy security have been the main driving forces for the development of renewable fuels that can replace the petroleum-derived fuels, such as gasoline and diesel. Ethanol is currently the most commonly used renewable automobile fuel. It is largely produced by fermentation of cellulosic biomass, such as sugar- or starch-containing feedstocks. However, the supply of these crops is relatively limited, and many of them can be considered as a human food resource. Cellulosic biomass comprising lignin (lignocellulosic biomass), on the other hand, is a more abundant and less expensive raw material with the potential to give a higher net energy gain. In lignocellulose, cellulose and hemicellulose are tightly associated to lignin, a polyphenolic compound that ties the cellulose and hemicellulose polymers together, thus providing the lignocellulose with rigidity and mechanical strength.

In the production of ethanol from cellulosic biomass, various pretreatment and hydrolysis steps are generally used to degrade the cellulose and hemicellulose polysaccharides to fermentable saccharides. The fermentable saccharides are then converted to ethanol, usually by fermentation with microorganisms such as yeast, and the ethanol is recovered by means of distillation.

After steaming and impregnation of the cellulosic biomass with various acids, hydrolysis at elevated temperature results in a slurry of solid and liquid cellulose-derived material. The acid catalysed hydrolysis might either be in two steps for degradation of both hemicellulose and cellulose polymers, or a milder pretreatment with hydrolysis of hemicellulose followed by an enzymatic treatment, to further degrade the cellulose polymers.

To facilitate a good fermentation of a hydrolysate obtained from acidic hydrolysis, the slurry must be filtered and washed, typically through a filter press, to remove the solid fraction of the slurry. However, the filtering and subsequent washing may result in a loss of fermentable saccharides. To optimize the washing sequence and minimize the sugar losses, a large amount of process water is required, leading to a dilution of the hydrolysate. A diluted hydrolysate in turn entails a higher distillation cost. Further, fermentation of the hydrolysate without removing the solid fraction is not an optimal solution, since the presence of solid material prevents recirculation of fermenting microorganisms, thus requiring new fermenting organisms to be continuously cultured, which is laborious and costly.

Some of the problems associated with acidic hydrolysis may be overcome by using a SSF (simultaneous saccharification and fermentation) process, in which enzymes capable of hydrolysing polysaccharides and the fermenting microorganism are directly mixed with the slurry. However, also in this case, the fermenting organisms can not be recirculated, which has the negative consequences as explained above. Further, the temperature of the enzymatic hydrolysis and the fermentation process has to be the same, which may not be optimal for either of the two reactions. Moreover, SSF requires intensive stirring and cooling of the slurry, which adds to the complexity of the process.

### Summary of the disclosure

To summarize, there is a need in the art for an improved fermenting method to produce ethanol from cellulosic and lignocellulosic biomass.

It is an aim of the present disclosure to provide for the preparation of a target chemical, such as ethanol, from a cellulose-derived slurry.

Further, it is an aim of some aspects of the present disclosure to provide for an energy-efficient production of the target chemical.

A further aim of some aspects of the present disclosure is to provide for improved process economy in the production of the target chemical..

A non-limiting and itemized listing of embodiments of the present disclosure is presented below for the purpose of providing various features and combinations provided by the invention in certain of its aspects.

### ITEMS

1. A method for providing a target chemical, comprising:
   a) providing a cellulose-derived slurry comprising fermentable saccharides in a container;
   b) separating a liquid fraction comprising part of said fermentable saccharides from said cellulose-derived slurry;
   c) transferring said liquid fraction to a fermentor;
   d) fermenting fermentable saccharides in said liquid fraction in said fermentor to provide a fermented liquid comprising said target chemical; and
   e) returning said fermented liquid to said container.
2. The method according to item 1, wherein the separating of step b) is performed in a separator providing said liquid fraction and a suspended solids fraction, which is returned to said container.
3. The method according to item 1 or 2, wherein said separating of step b) comprises filtering or decanting.
4. The method according to any previous item, wherein the target chemical is recovered from the fermented liquid during step e).
5. The method according to any one of items 1-3, wherein the target chemical is recovered from said container.
6. The method according to item 5, wherein the temperature in said container is at least 70 °C.
7. The method according to any one of items 1-3, further comprising:
   f) recovering the target chemical from at least part of the content of said container in a target recovery unit.
8. The method according to any one of items 4-7, wherein said recovery of said target chemical is performed by means of distillation.
9. The method according to any previous item, wherein said target chemical is selected from the group consisting of ethanol, butanol and succinic acid.
10. The method according to any previous item, wherein step d) comprises contacting said liquid fraction with a fermenting organism to obtain said fermentation liquid.
11. The method according to item 10, wherein said fermenting organism is
   retained in said fermentor.
12.The method according to item 11, wherein said fermenting organism is
   retained by means of sedimentation, centrifugation or immobilization.
13. The method according to item 10-12, wherein said target chemical is
   ethanol and said fermenting organism is a yeast.
14.The method according to any previous item, wherein said cellulose-
   derived slurry of step a) is obtained by a method comprising:
   a1) providing optionally pretreated cellulosic biomass; and
   b1) subjecting said cellulosic biomass from step a1) to hydrolysis
      in at least one hydrolysis unit separate from said container to obtain said cellulose-derived slurry.
15.The method according to item 14, wherein said hydrolysis is a two-
   step acidic hydrolysis.
16. The method according to any one of items 1-13, wherein said cellulose-derived slurry of step a) is obtained by a method comprising the steps of:
   a2) providing optionally pretreated cellulosic biomass; and
   b2) subjecting said cellulosic biomass to enzymatic hydrolysis in
      said container to obtain said cellulose-derived slurry.
17.The method according to item 16, wherein saccharification enzymes
   are used in step b2) and said saccharification enzymes are further recovered from said container and reused.
18.The method according to any one of items 1-17, wherein the
   temperature of said slurry in said container is between 30 and 100°C, such as between 40 and 70°C, and the temperature of said liquid fraction in said fermentor is between 25 and 90°C, such as between 28 and 38 °C.
19. The method according to any one of items 14-18, wherein said cellulosic biomass is lignocellulosic biomass, such as sugarcane bagass or wood.
20. The method according to any previous item, wherein step e) comprises heating said fermentation liquid by bringing it into thermal contact with said liquid fraction of step c).
21.The method according to item 20, wherein said thermal contact is
   achieved in a heat exchanger.
22. The method according to any one of the previous items, wherein the transferring of c) and/or the returning of e) is/are performed by means of pumping.
23. Use of a fermented, cellulose-derived hydrolysate for extracting fermentable saccharides from a cellulose-derived slurry, wherein said cellulose-derived slurry has a suspended solids content of from 5 % to about 30 %.
24.A system for fermenting sugars to a target chemical, comprising:
   a container for containing a cellulose-derived slurry comprising fermentable saccharides;
   a separator for separating a liquid fraction comprising fermentable saccharides from said slurry, which separator is connected to said container;
   a fermentor for fermenting at least part of said fermentable saccharides in said liquid fraction to yield a fermented liquid, which fermentor comprises an inlet and an outlet;
   a first connection connecting said separator and said fermentor inlet and
   a second connection connecting said fermentor outlet and said container.
25. The system according to item 24, wherein said first connection connecting said separator and said fermentor is a first transferring arrangement for transferring said liquid fraction from said separator to said fermentor and said second connection is a second transferring arrangement for transferring said fermented liquid from said fermentor to said container.
26. The system according to item 25, further comprising
   a first pump for pumping said liquid fraction, which pump is arranged on said first transferring arrangement and/or
   a second pump for pumping said fermented liquid, which second pump is arranged on said second transferring arrangement.
27. The system according to item 25 or 26, wherein
   said separator comprises a slurry inlet, a liquid fraction outlet and a suspended solids fraction outlet,
   said system further comprises a third transferring arrangement for transferring at least part of said slurry from said container to said separator and a fourth transferring arrangement for returning a suspended solids fraction from said separator to said container,
   said first transferring arrangement is connected to said liquid fraction outlet of said separator,
   said third transferring arrangement is connected to said container and said slurry inlet of said separator and
   said fourth transferring arrangement is connected to said solids fraction outlet of said separator and said container.
28. The system according to items 27, further comprising a third pump for pumping said slurry, which pump is arranged on said third transferring arrangement.
29. The system according to any one of items 25-28, wherein the separator comprises at least one filter, decanter or centrifuge.
30. The system according to any one of items 25-29, further comprising a heat exchanger for transferring heat from said liquid fraction to said fermented liquid, which heat exchanger is arranged on said first transferring arrangement and said second transferring arrangement.
31.The system according to item 30 comprising said first pump and said
   second pump, wherein said first pump is arranged upstream of said heat exchanger on said first transferring arrangement and said second pump is arranged upstream of said heat exchanger on said second transferring arrangement.
32. The system according to any one of items 24-31, wherein said container is provided with a heater.
33. The system according to any one of items 24-32, further comprising a target chemical recovery apparatus arranged on said second transferring arrangement.
34.The system according to item 33, wherein the target chemical
   recovery apparatus comprises at least one distillation unit.
35. The system according to any one of items 24-32, wherein said container is adapted for recovery of said target chemical from said container.
36. The system according to item 35, wherein distillation means is arranged on said container.
37. The system according to item 36, wherein said distillation means comprises a distillation column.
38. The system according to any one of items 25-32, further comprising a target recovery unit for recovering the target chemical from at least part of the content of the container, which target recovery unit is connected to said container.
39. The system according to any one of items 25-38, wherein said fermentor is provided with a cooler.
40. The system according to any one of items 24-39, wherein said fermentor is adapted for retaining a fermenting organism provided in said fermentor during fermentation.
41.The system according to item 40, wherein said fermentor is adapted to
   retain yeast.
42. The system according to any one of items 25-41, further comprising at least one hydrolysis unit for hydrolyzing cellulosic biomass and providing said cellulose-derived slurry, which at least one hydrolysis unit is connected to said container.
43. The system according to any one of items 25-41, further comprising an enzyme recovery arrangement connected to said container, for recovering enzymes from said container.
44. A system for withdrawing a liquid comprising a target chemical from a fermentor while retaining a fermenting agent inside said fermentor, said system comprising:
   a fermentor for holding a liquid comprising said fermenting agent and said target chemical;
   at least one pipe having a first end below the surface of said
   liquid in said fermentor and a second end above said surface;
      wherein the diameter of said at least one pipe is configured so that an under pressure applied to said second end of said at least one pipe causes liquid comprising said target chemical to rise from said first to said second end of said at least one pipe at a rate that is lower than the average settling rate of said fermenting agent,
      wherein liquid comprising said target chemical is withdrawn from said second end of said at least one pipe and said fermenting agent is retained in said fermentor.
45. A method for withdrawing a liquid comprising a target chemical from a fermentor while retaining a fermenting agent inside said fermentor comprising the steps of:
   a) providing a liquid comprising said target chemical and fermenting agent in a fermentor, said fermentor comprising at least one pipe having a first end below the surface of said liquid in said fermentor and a second end above said surface;
   b) applying an under pressure to said second end of said at least one pipe so that liquid comprising said target chemical rises from said first to said second end of said at least one pipe, wherein the diameter of said at least one pipe is configured so that said liquid comprising said target chemical rises at a rate that is lower than the average settling rate of said fermenting agent, wherein liquid comprising said target chemical is withdrawn from said second end of said at least one pipe and said fermenting agent is retained in said fermentor.

### Detailed description

In the present disclosure, there is provided a method for providing a target chemical, comprising:
a) providing a cellulose-derived slurry comprising fermentable saccharides in a container;
b) separating a liquid fraction comprising part of the fermentable saccharides from the cellulose-derived slurry;
c) transferring the liquid fraction to a fermentor;
d) fermenting fermentable saccharides in the liquid fraction in the fermentor to provide a fermented liquid comprising the target chemical; and
e) returning the fermented liquid to the container.

A "target chemical" refers to a chemical of interest that can be prepared by fermentation of fermentable saccharides.

A "slurry" refers to a semifluid suspension of solids in a liquid, i.e. solid material suspended in a liquid. A cellulose-derived slurry is thus a slurry wherein the solids content has cellulosic origin.

"Fermentable saccharides", such as fermentable monosaccharides and disaccharides, refer to sugars that can be converted, e.g. fermented, into a target chemical, e.g. by a microbiological fermenting agent. Fermentable monosaccharides may comprise simple sugars with different numbers of carbon atoms, such as pentoses having five carbon atoms, hexoses having six carbon atoms and heptoses having seven carbon atoms. An example of a fermentable disaccharide is fructose. In general, which saccharides to be considered fermentable saccharides depends on the fermenting agent(s) employed. That is, the fermentable saccharides are those which may be fermented by the fermenting agent(s).

A "container" refers to any construction capable of holding the material in question, and it may be comprised of different materials, such as metals, stainless steel etc.

Separating a liquid fraction comprising part of the fermentable saccharides from the cellulose-derived slurry refers to dividing the cellulose-derived slurry into different fractions, wherein at least one fraction is liquid, i.e. has a low content of suspended solids, and which liquid fraction comprises at least a part of the fermentable saccharides. The liquid fraction has in any case a content of suspended solids which is lower than that of the cellulose-derived slurry. Preferably, suspended solids content is below 5 g/l, such as below 2 g/l.

Fermenting fermentable saccharides refers to converting the fermentable saccharides to the target chemical by means of fermentation. Fermentation is a process known to the skilled person, and is usually performed by microorganisms.

A "fermentor" refers to a container suitable for the fermentation reaction in question.

Further, in the present disclosure there is provided a system for fermenting sugars to a target chemical, comprising:
a container for containing a cellulose-derived slurry comprising fermentable saccharides;
a separator for separating a liquid fraction comprising fermentable saccharides from the slurry, which separator is connected to the container;
a fermentor for fermenting at least part of the fermentable saccharides in the liquid fraction to yield a fermented liquid, which fermentor comprises an inlet and an outlet;
a first connection connecting the separator and the fermentor inlet and a second connection connecting the fermentor outlet and the container.

A "connection" refers to any type of connection through which a liquid can pass from one unit to another. As an example, a connection may be an interface provided with a membrane or a mesh-like structure.

The method and system aspects according to the present disclosure are based on the insight that a fermentation for providing a target chemical from a cellulose-derived slurry may be performed by separating a liquid fraction from the cellulose-derived slurry, fermenting the separated liquid and returning the liquid from the fermentation to the cellulose-derived slurry, and that such a fermentation has a number of positive effects.

If the disclosed method and system are used after acid hydrolysis of cellulosic biomass, washing of the slurry with additional water may be substantially reduced or avoided. By separating a liquid fraction from the slurry and then returning the fermented liquid, the slurry is instead washed with the fermented liquid, facilitating liberation of monosaccharides from the solid content of the slurry. Thus, less external process water is needed, which in turn leads to less dilution of the target chemical and facilitates an efficient recovery of the target chemical.

Moreover, if the disclosed method and system are used together with enzymatic hydrolysis of a cellulose-derived slurry, the temperature during the enzymatic hydrolysis in the container and the temperature during fermentation in the fermentor may be kept different, since the container is separate from the fermentor. This facilitates favorable conditions for both the hydrolysis reaction and the fermentation reaction if the two reactions have different optimal temperatures. As an example, the temperature during enzymatic hydrolysis may be kept higher than to the temperature during fermentation, thus facilitating the use of thermophilic enzymes.

Further, the method and system according to the present disclosure do not require intensive stirring and cooling of the slurry, since the fermentation is performed in a separate fermentor. The method and system according to the present disclosure also facilitate the use of a higher temperature of the slurry in the container, which lowers the viscosity of the slurry, thereby making it possible to use a higher suspended solids content in the slurry. Moreover, if microorganisms are used for fermenting the monosaccharides, the microorganisms may be reused in several fermentation cycles in the fermentor, which in turn decreases the need for culturing new microorganisms. A fermentation of a slurry does normally not allow for such reuse of the fermenting agent because the solids of the slurry and the fermenting agent may not be separated.

The process in the slurry container may be performed batch-wise, but it may also be referred to as a fedbatch process if the container is filled up slowly. The latter may be advantageous since it allows for a gradual digestion of the slurry and a lower concentration of solids in the slurry container than in the feed, which facilitates stirring, pumping and filtration. After being filled up, the slurry container may be kept at a constant volume while separated liquid is circulated through the fermentation vessel and back again. In batch and fedbatch process, the container is finally emptied and then normally refilled with fresh slurry. How fast the slurry vessel is filled up and how long it is kept at constant volume is a matter of optimization. Filling and emptying of the slurry vessel may also be performed continuously.

The preparation of the target chemical from the cellulose-derived slurry is preferably performed as a continuous process, wherein a sugar-containing liquid fraction is constantly separated from one or several slurry containers and pumped through the fermentation vessel, in which a culture of fermenting organisms is retained. A stream with higher content of the target chemical but lower sugar concentration is fed back to the slurry container or containers. The feed is however interrupted when the slurry container is emptied or refilled. In principle, the feeding pattern may also be altered so that it fits the definition of a batch or fedbatch process, i.e., the fermentation may be performed batch-wise.

A certain time may be required to reach a sufficient target chemical concentration in the system of the present disclosure or to consume a sufficient amount of the saccharide material. Accordingly, the method of the first aspect of the invention may be performed for at least 1 hour, such as for at least 12 hours, such as for at least 18 hours, such as for at least 24 hours. Thus, the steps of separating of a liquid fraction from the container, transferring the liquid fraction to the fermentor, fermenting fermentable saccharides in the fermentor and returning a fermented liquid may be performed for at least 1 hour, such as for at least 12 hours, such as for at least 18 hours, such as for at least 24 hours.

As discussed above, several containers for containing cellulose-derived slurries may feed a single fermentor with a liquid fraction, and a fermented liquid from a single fermentor may be returned to several containers.

In an embodiment of the system aspect of the disclosure, the first connection connecting the separator and the fermentor is a first transferring arrangement for transferring the liquid fraction from the separator to the fermentor and the second connection is a second transferring arrangement for transferring the fermented liquid from the fermentor to the container. The first and/or second transferring arrangement may for example comprise one or several pipes.

In an embodiment of the method aspect of the disclosure, the separating of step b) is performed in a separator providing the liquid fraction and a suspended solids fraction, which is returned to the container. A "suspended solids fraction" refers to a fraction having a suspended solids content that is higher than the suspended solids content of the cellulose-derived slurry in the container. For example, the suspended solids content of the suspended solids fraction is 5 g/l or higher, such as 10 g/l or higher. Returning the suspended solids fraction to the container allows for optimal use of fermentable saccharides that are still contained in the suspended solids fraction, i.e. fermentable saccharides that are not comprised in the liquid fraction but for example are embedded in or adsorbed to the solid content

In an embodiment of the system aspect of the disclosure, in which the first and second connections are a first and second transferring arrangement, respectively,
the separator comprises a slurry inlet, a liquid fraction outlet and a suspended solids fraction outlet,
the system further comprises a third transferring arrangement for transferring at least part of the slurry from the container to the separator and a fourth transferring arrangement for returning a suspended solids fraction from the separator to the container,
the first transferring arrangement is connected to the liquid fraction outlet of the separator,
the third transferring arrangement is connected to the container and the slurry inlet of the separator and
the fourth transferring arrangement is connected to the solids fraction outlet of the separator and the container.

In an embodiment of the method aspect of the invention, the transferring of c) and/or the returning of e) is/are performed by means of pumping.

Pumping is a convenient way of actively transferring liquids in the system. Further, if the separating of step b) is performed in a separator, a transporting arrangement such as a moving belt may be used to return the suspended solids fraction to the container.

In an embodiment of the system aspect of the disclosure, in which the first and second connection are a first and second transferring arrangement, respectively, the system further comprises a first pump for pumping the liquid fraction, which pump is arranged on the first transferring arrangement and/or a second pump for pumping the fermented liquid, which second pump is arranged on the second transferring arrangement.

The system may also comprise a third pump for pumping the slurry, which pump is arranged on the third transferring arrangement.

In another embodiment of the method aspect, the separating of step b) comprises filtering or decanting. Filtering refers to the use of one or several types of filters or grid-like structures for removing solids from the liquid, thus providing a liquid fraction substantially free of solids. Decanting refers to the removal of a clear supernatant liquid from a sediment, e.g. after centrifugation. Filtering or decanting are convenient ways for efficiently separating a liquid fraction from a slurry.

In an embodiment of the system aspect of the invention, the separator comprises at least one filter, decanter or centrifuge.

The at least one filter may be at least one filter of the same type or two or more filters of different types, such as filters of different pore sizes or structures for achieving a desired separation effect. The at least one filter may be at least one band filter, drum filter, disc filter and/or at least one membrane.

In another embodiment of the first aspect, the target chemical is recovered from the fermented liquid during step e).

Recovering the target chemical from the fermented liquid refers to removing at least part of the target chemical from the fermented liquid.

The target chemical may continuously be recovered during step e) or the target chemical may be recovered from step e) after a certain concentration of target chemical has been built up in the system.

In an embodiment of the system aspect of the disclosure, in which the first and second connections are a first and second transferring arrangement, respectively, the system further comprises a target chemical recovery apparatus arranged on the second transferring arrangement. A target chemical recovery apparatus refers to an apparatus adapted for recovery of the target chemical. In such an apparatus, heat and/or vacuum may be employed to drive off the target chemical.

In an embodiment of the method aspect of the disclosure, the target chemical is recovered from the container.

In an embodiment of the system aspect of the disclosure, the container is adapted for recovery of the target chemical from the container.

Thus, the target chemical may be recovered directly from the container, without withdrawing a content from or emptying the container. If the target chemical is recovered from the container, the temperature in the container may be at least 70 °C. If the temperature of the container is higher than the boiling point of the target chemical, the target chemical may evaporate in the container, which facilitates recovery of the target chemical directly from the container by means of e.g. distillation.

In another embodiment of the first aspect, the method further comprises the step:
f) recovering the target chemical from at least part of the content of the container in a target recovery unit.

In an embodiment of the system aspect of the disclosure, the system further comprises a target recovery unit for recovering the target chemical from at least part of the content of the container, which target recovery unit is connected to the container.

A target recovery unit refers to a unit adapted for recovering the target chemical.

Recovering the target chemical from the container may be performed by withdrawing at least part of the content of the container and recover the target chemical from that part or emptying substantially the whole content of the container before recovering the target chemical. The part of the container content from which the target chemical is to be recovered or the whole container content may also be filtered before recovering the target chemical.

In an embodiment of the method aspect, the recovery of the target chemical is performed by means of distillation. Distillation of the target chemical refers to vaporization of the target chemical, followed by cooling and condensing the vapor of the target chemical and collecting the resulting liquid of the target chemical.

In an embodiment of the system aspect, in which a target chemical recovery apparatus is arranged on the second transferring arrangement, the target chemical recovery apparatus comprises at least one distillation unit. The distillation unit may comprise distillation means, such as at least one distillation column.

In an embodiment of the system aspect, in which the target chemical is recovered from the container, distillation means are arranged on the container. The distillation means may comprise a distillation column.

In another embodiment of the system aspect, in which the system comprises a target recovery unit connected to the container, the target recovery unit comprises distillation means, such as at least one distillation column.

In another embodiment of the method aspect, the target chemical is recovered by means of extraction and/or a process employing at least one membrane. If the target chemical is e.g. succinic acid, an extraction process and/or a process employing at least one membrane may be favorable. In an embodiment of the system aspect of the disclosure, the target chemical recovery apparatus arranged on the second transferring arrangement and/or the target recovery unit comprises at least one membrane.

In an embodiment of the method aspect, the target chemical is selected from the group consisting of ethanol, butanol and succinic acid. These target chemicals derivable from cellulose can be produced from fermentable saccharides by means of fermentation. Other examples of target chemicals may be other alcohols or acids, alkanes, alkenes, aromatics, aldehydes, ketones, biopolymers, proteins, peptides, amino acids, vitamins, antibiotics and other pharmaceuticals.

In an embodiment of the method aspect, step d) comprises contacting the liquid fraction with a fermenting organism to obtain the fermentation liquid.

A fermenting organism refers to an organism capable of fermenting fermentable saccharides into a target chemical. Contacting the liquid fraction with the fermenting organism may be achieved by using a fermentor containing the fermenting organism. The fermenting organism may be at least one eukaryotic or prokaryotic microorganism, such as bacteria and/or yeast. Bacteria and yeasts are known to be capable of fermenting saccharides into other chemical compounds. Yeasts from *Saccharomyces, Pichia* and *Candida* may be used as the fermenting organism. The fermenting organism may for example be wild type, mutant or recombinant *Saccharomyces cerevisiae.*

*S. cerevisiae* is one of the most suitable microorganisms for ethanol production from hexose sugars and is favored in industrial processes. Further, the fermenting organism may be wildtype, mutant or recombinant *Escherichia coli.* Recombinant *E. coli* strains may be useful when the target chemical is e.g. butanol or succinic acid. In an embodiment of the method aspect, the target chemical is ethanol and the fermenting organism is a yeast.

In an embodiment of the method aspect, the fermenting organism is retained in the fermentor. Retaining the fermenting organism in the fermentor refers to designing the fermentor or the fermenting conditions such that the amount of the fermenting organism does not substantially decrease in the fermentor when the fermented liquid is returned to the container. Retaining the fermenting organism may allow fermenting organisms to leave the fermentor if the growth of the fermenting organism in the fermentor compensates for the loss. Further, retaining the fermenting organism in the fermentor may allow the fermenting organism to leave the fermentor but be returned by the use of a fermenting organism returning system arranged downstream from the fermentor. Retaining the fermenting organism in the fermentor thus embodies the reuse of the fermenting organism in several cycles in the method according to the present disclosure, e.g. if step b) - e) is repeated. Consequently, fresh fermenting organisms do not need to be cultured for every cycle. Further, in another embodiment of the method aspect, fresh fermenting organisms are added to the fermentor while at least some of the fermenting organisms are retained in the fermentor. This means that fresh fermenting organisms may be continuously added to the fermentor or added to the fermentor between cycles of the method according to the present disclosure, e.g. in order to ensure a high fermenting activity in the fermentor.

As an example, the fermenting organism may be retained by means of sedimentation, centrifugation or immobilization.

Sedimentation refers to allowing the fermenting agent to settle towards the bottom of a separate vessel and then pumping the settled fermenting agent back into the fermentor, while clear liquid is withdrawn from the top of the separate vessel.

Centrifugation refers to the use of rotation in a separate device in order to create a centrifugal force so that more dense components migrate away from the axis of the device. Thus, the fermenting agents (dense components) may be separated from the liquid and returned to the fermentor.

Further, immobilization refers to entrapment of the fermenting agent in the fermentor, such as entrapment within a porous but solid-like material which either moves freely in the fermentation liquid or is attached to the walls of the fermentor. Alternatively, other structures present in the fermentor may be used for the entrapment. For example, the fermenting agent may be entrapped by means of encapsulation in beads in the fermentor.

In some fermentation processes, the biomass (the fermentation agent) tends to leave the fermentor at a higher rate than the rate of which it is reproduced. This is sometimes referred to as "wash out". The risk of a wash out is particularly high when the fermentable liquid contains substances which inhibit the fermenting agent. If the wash out cannot be prevented, the volume of the fermentor may have to be increased, which entails costs, or the supply of fermentable liquid has to be decreased, which lower the capacity of the system.

The wash out problem may be solved using various cell retention techniques, such as immobilization, or recirculation by centrifugation. This is further explained above. Alternatively, a settler may be employed. In such case, the liquid-fermenting agent mixture from the fermentor is pumped to an external vessel in which the fermenting agent settles (sinks) and is pumped back to the fermentor (see above). Normally, in such an arrangement, the fermenting agent has to flocculate to obtain a sufficient settling rate. Drawbacks of an external settler are the investment in an additional vessel and the increased pumping capacity needed. Further, the external settler arrangement may cause operation interruptions due to clogging of the settler.

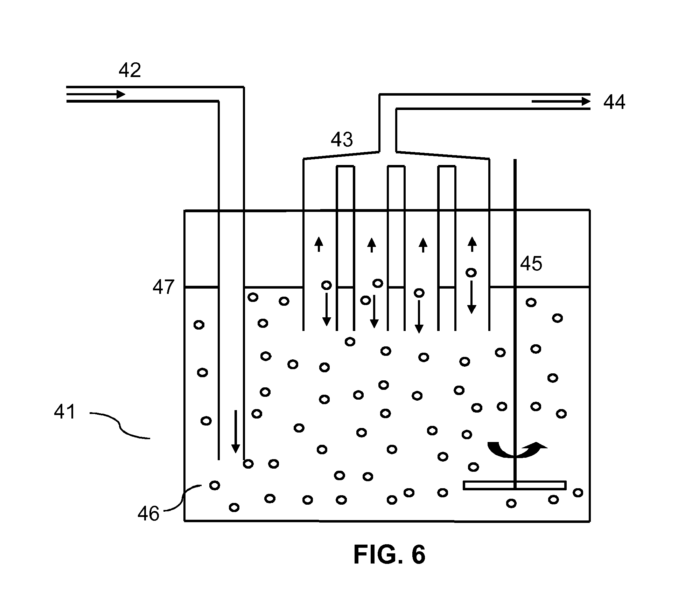

Thus, it might be beneficial to use an internal settler according to item 44. One embodiment of such an arrangement, which is an integrated fermentation vessel and settler, is shown in Figure 6. In Figure 6, the outgoing flow is sucked up in one or more pipes. The number and dimensions of the pipes are such that the upward flow rate is lower than the average settling rate of the fermenting agent. The settler is preferably provided with an agitator, and the agitation during operation is preferably such that the turbulence by the inlet of the pipes is kept low.

The fermented liquid (containing the target chemical) is thus sucked from the surface region of the liquid of the fermentor while the fermentation agent is retained to a large extent.

As with an external settler, flocculation with of the fermenting organism may be necessary for operation, i.e., to obtain a sufficient settling rate.

For example, a flocculating fermenting agent (such as a flocculating yeast) may be used or a flocculation agent may be added.

The use of an internal settler as described above and according to item 44 may also reduce the risk of infection in the fermentor, since the flow-rate in the fermentor provides a "wash out" for everything that has a lower settling rate than the average rate at which liquid rises in the pipes, including infectious organisms, such as bacteria. Consequently, a high turnover of liquid in the fermentor may rinse out infectious organisms from the fermentor, which decreases the risk of an infection in the fermentor. Further, if the internal settler is used in a system where the fermented liquid withdrawn from the fermentor is returned to a zone having an elevated temperature, such as the container of the present disclosure, the infectious organisms from the fermentor may be killed or inactivated to a large extent, thus decreasing the concentration of infectious organisms in the whole system.

In an embodiment of the system aspect of the disclosure, the fermentor is adapted for retaining a fermenting organism provided in the fermentor during fermentation. As an example, the fermentor may be adapted to retain yeast. The fermentor may be adapted for retaining a fermenting organism e.g. by having an "internal settler" as described above.

In an embodiment of the method aspect of the disclosure, the cellulose-derived slurry of step a) is obtained by a method comprising:
a1) providing optionally pretreated cellulosic biomass; and
b1) subjecting the cellulosic biomass from step a1) to hydrolysis in at least one hydrolysis unit separate from the container to obtain the cellulose-derived slurry.

In such an embodiment, the cellulose-derived slurry is preferably neutralized before it is added to the container. The neutralization may for example be performed by means of an addition of a base, such as NaOH or lime stone.

As an example, the cellulosic biomass may be lignocellulosic biomass, such as sugarcane bagass or wood. Lignocellulosic biomass refers to biomass that comprises cellulose, lignin and possibly hemicellulose. The lignocellulosic biomass may for example be wood residues or forestry residues, such as wood chips, sawmill or paper mill discards, or agricultural residues. Lignocellulosic biomass is an abundant raw material with the potential to give a high net energy gain and has a high renewable CO₂ efficiency.

"Pretreated cellulosic biomass" refers to cellulosic biomass that has been pretreated in order to modify its properties such that the cellulose become more accessible during subsequent hydrolysis. As an example, the pretreatment may decrease the degree of crystallinity of the cellulosic biomass and/or the porosity of the cellulosic biomass. The pretreatment may involve one or several pretreatment methods known to the skilled man. As an example, the pretreatment may be impregnation. Impregnation refers to impregnating the cellulosic biomass with an impregnation fluid. The fluid may be an acid solution, such as a mineral acid solution. The impregnation may also be performed with a gas, such as a SO₂-gas, or with the combination of a gas with a liquid. The pretreatment may also be steaming or steaming followed by impregnation. Steaming refers to a process used to drive air out from the cellulosic biomass to facilitate further hydrolysis of the cellulose. Steaming is a well-known method for pretreating e.g. lignocellulosic biomass. As other examples, pretreatment may involve prehydrolysis, impregnation, steaming, steam explosion or any combination thereof. Steam explosion refers to a process that combines steam, shearing forces and hydrolysis for rupturing cellulosic fibers. Subjecting the cellulosic biomass from step a1) to hydrolysis refers to subjecting the cellulose of the cellulosic biomass to a chemical breakdown due to reaction with water. As an example, the hydrolysis may be a two-step acidic hydrolysis. A two-step acidic hydrolysis refers to a hydrolysis of the optionally pretreated cellulosic biomass in two steps in an acidic environment, such as in an environment that has a pH of below 4. The acidic environment may be achieved by the addition of at least one mineral acid, such as sulfuric acid, hydrochloric acid, nitric acid, phosphoric acid, boric acid and/or hydrofluoric acid. Further, at least one hydrolysis step of the two-step acid hydrolysis may be performed at a pressure that is higher than atmospheric pressure. The two hydrolysis steps may be performed in two separate hydrolysis units and in two different acid environments, which may be achieved by addition of two different acids in the two hydrolysis units or by using different pressures in the two separate hydrolysis units. The acids used in the two-step acidic hydrolysis may be diluted acids.

In an embodiment of the system aspect of the invention, the system further comprises at least one hydrolysis unit for hydrolyzing cellulosic biomass and providing the cellulose-derived slurry, which at least one hydrolysis unit is connected to the container.

The at least one hydrolysis unit may be adapted for a two-step acidic hydrolysis according to the above. The at least one hydrolysis unit may comprise stainless steel.

In another embodiment of the method aspect of the disclosure, the cellulose-derived slurry of step a) is obtained by a method comprising the steps of:
a2) providing optionally pretreated cellulosic biomass; and
b2) subjecting the cellulosic biomass to enzymatic hydrolysis in the container to obtain the cellulose-derived slurry.

Regarding "pretreated cellulosic biomass", see above. A pretreated biomass is preferably neutralized before added to the container.

As an example, the cellulosic biomass may be lignocellulosic biomass, such as sugarcane bagass or wood. Lignocellulosic biomass and pretreated cellulosic biomass refers to as described above. Enzymatic hydrolysis refers to a hydrolysis reaction catalysed by at least one enzyme. The at least one enzyme may be at least one saccharification enzyme, which refers to at least one enzyme that can convert or hydrolyse cellulosic biomass into fermentable saccharides, such as monosaccharides and/or disaccharides. Such saccharification enzymes may be glycosidases, which hydrolyse polysaccharides. Examples of glycosidases include cellulose-hydrolysing glycosidases, such as cellulases, endoglucanases, exoglucanases, cellobiohydrolases and β-glucosidases, hemicellulose hydrolysing glycosidases, such as xylanases, endoxylanases, exoxylanases, β-xylosidases, arabinoxylanases, mannases galactases, pectinases and glucuronases, and starch hydrolysing glycosidases, such as amylases, α-amylases, β-amylases, glucoamylases, α-glucosidases and isoamylases, or any enzymes in the group of enzymes found in EC 3.2.1.x, such as EC 3.2.1.4, where EC is the Enzyme Commission number.

As an example, saccharification enzymes may be used in step b2) and the saccharification enzymes may be further recovered from the container and reused.

Recovering and reusing the enzymes from the container may reduce the use of or need for external enzymes when hydrolysing cellulosic biomass. Enzymes may be recovered from contents that are withdrawn from the container. If slurry is continuously withdrawn from the container, the enzymes may continuously be recovered from the container. If the container is emptied after a certain time of processing or when a certain target chemical concentration is reached, enzymes may be recovered from the material that was emptied from the container.

Recovered enzymes may then be reused in step b2) to obtain cellulose-derived slurry from new cellulosic biomass.

In an embodiment of the system aspect of the disclosure, the system further comprises an enzyme recovery arrangement connected to the container, for recovering enzymes from the container.

The enzyme recovery arrangement may comprise purification means for recovering the enzymes, such as at least one affinity system or affinity chromatography column with affinity for the enzyme to be recovered.

In an embodiment of the method aspect of the invention, the temperature of the slurry in the container is between 30 and 100°C, such as between 40 and 70°C, and the temperature of the liquid fraction in the fermentor is between 25 and 90°C, such as between 28 and 38 °C.

The temperatures of the slurry in the container and the liquid fraction in the fermentor may thus be different. The different temperatures may facilitate good conditions both for the slurry in the container and the fermentation in the fermentor. As an example, if an enzymatic hydrolysis is performed in the container in step b2), the temperature of the enzymatic hydrolysis reaction in the container may have a different optimal temperature compared to the optimal temperature when fermenting fermentable saccharides in the fermentor. As an example, if a yeast is used as the fermenting organism in the fermentor and enzymatic hydrolysis is used in step b2), the temperature of the slurry in the container may be between 60 and 90°C and the temperature of the fermenting liquid in the fermentor may be about 35°C. Further, if a thermophilic microorganism is used as a fermenting organism in the fermentor, the optimal temperature of the liquid in the fermentor may be between 60 and 90°C. As a further example, if the cellulosic biomass is obtained by steps a1) and b1), the temperature of the slurry in the container and the temperature of the fermenting liquid in the fermentor may be similar, such as around 35°C.

In an embodiment of the system aspect of the disclosure, the container is provided with a heater.

A heater facilitates increasing of the temperature of the container if for example the cellulose-derived slurry provided in the container and/or the fermented liquid that is transferred to the container has a lower temperature than what is desired. If for example an enzymatic hydrolysis is performed in the container, such as in step b2) described above, the enzymatic hydrolysis reaction may have a higher optimal temperature, i.e. a higher temperature at which a maximum number of polysaccharides are hydrolysed by the reaction catalyzed by the enzyme, than the temperature of the cellulose-derived slurry and/or the temperature of the fermented liquid. Thus, a heater may facilitate the use of a different temperature in the container compared to e.g. the fermentor.

In another embodiment of the system aspect of the disclosure, the fermentor is provided with a cooler.

If the fermentor is provided with a cooler, it facilitates decreasing of the temperature in the fermentor if the liquid fraction transferred to the fermentor has a higher temperature than what is favorable for the fermentation reaction.

In an embodiment of the method aspect of the invention, step e) comprises heating the fermentation liquid by bringing it into thermal contact with the liquid fraction of step c). Bringing the fermentation liquid into thermal contact with the liquid fraction refers to subjecting the fermentation liquid to conditions where it can exchange energy in the form of heat with the liquid fraction. As an example, the thermal contact may be achieved in a heat exchanger. Bringing the fermentation liquid into thermal contact with the liquid fraction e.g. in a heat exchanger is an efficient way of passively exchanging heat between the fermentation liquid and the liquid fraction. Thus, if the temperature in the container is higher than the optimal temperature of the fermentor, e.g. if an enzymatic hydrolysis as in step b2) is performed in the container, heating the liquid that is transferred to the container with the liquid that is transferred to the fermentor, i.e. exchanging heat between the liquid fraction of step c) and the fermented liquid of step e), is an efficient way to preserve the energy in the method and system of the present disclosure. Such heat exchanging also reduces the need for cooling of the fermentor and/or heating of the container if different temperatures are used in the fermentor and container, respectively.

In an embodiment of the system aspect of the disclosure, in which the first and second connections are a first and second transferring arrangement, respectively, the system further comprises a heat exchanger for transferring heat from the liquid fraction to the fermented liquid, which heat exchanger is arranged on the first transferring arrangement and the second transferring arrangement. As an example, if the system further comprises the first and the second pump, the first pump may be arranged upstream of the heat exchanger on the first transferring arrangement and the second pump may be arranged upstream of the heat exchanger on the second transferring arrangement.

In a further aspect of the present disclosure, there is provided the use of a fermented, cellulose-derived hydrolysate for extracting fermentable saccharides from a cellulose-derived slurry, wherein the cellulose-derived slurry has a suspended solids content of from 5 % to 30 %, such as from 8 % to 25 %, such as from 12 % to 17 %.

The "fermented, cellulose-derived hydrolysate" has an ethanol content since it is fermented. Preferably, the cellulose-derived hydrolysate originates from the slurry from which it, in fermented form, is used to extract fermentable saccharides.

Methods for measuring a suspended solids content are known to a person skilled in the art. The use of a fermented, cellulose-derived hydrolysate for extracting fermentable saccharides from a cellulose-derived slurry may lead to less dilution of fermentable saccharides compared to if external process water is used.

### Brief description of the drawings

Figure 1 shows a schematic overview of a system according to an embodiment of the present disclosure, in which the cellulose-derived slurry is obtained by a two-step acidic hydrolysis.
Figure 2 shows a schematic overview of a system according to an embodiment of the present disclosure, in which the cellulose-derived slurry is obtained by an enzymatic hydrolysis.
Figure 3 shows a schematic overview of the experimental set-up used to prepare ethanol as a proof-of-concept of the present disclosure.
Figure 4 shows the result from Example 4. The ethanol, glucose and mannose concentrations in the hydrolysis vessel is plotted as a function of time. The x-axis represents the time in hours, and the y-axis represents the concentration in g/l. Filled diamonds represent ethanol, open triangles represent glucose and filled triangles represent mannose.
Figure 5 shows the result from Example 5. The ethanol, glucose and mannose concentrations in the hydrolysis vessel is plotted as a function of time. The x-axis represents the time in hours, and the y-axis represents the concentration in g/l. Filled diamonds represent ethanol, open triangles represent glucose and filled triangles represent mannose.
Figure 6 shows an example of a system for withdrawing a liquid comprising a target chemical from a fermentor while retaining a fermenting agent inside the fermentor.

### Detailed description of example embodiments

Non-limiting embodiments of the method and system of the present disclosure are described in more detail below.

### Example 1. Method and system for providing a target chemical, in which the cellulose-derived slurry is obtained by two-step acidic hydrolysis of cellulosic biomass

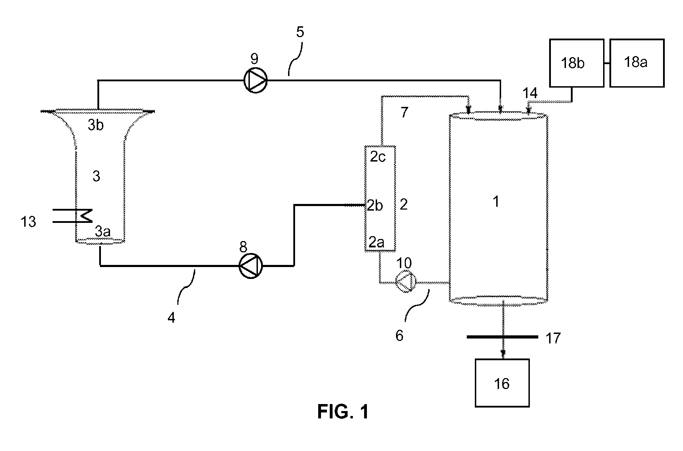

Figure 1 illustrates an embodiment of the disclosed method and system, in which the cellulose-derived slurry is obtained by a two-step acidic hydrolysis. The system for providing the target chemical comprises a container (1) for containing a cellulose-derived slurry. The cellulose-derived slurry, having a suspended solids content of about 15 %, is obtained by a two-step acidic hydrolysis performed in two hydrolysis units (18a and18b). Cellulosic biomass, pretreated e.g. by steaming, is provided in the first hydrolysis unit (18a), where it is subjected to conditions such as 170-190 °C and a pH 1.5-2.5. In the second step of the two-step acidic hydrolysis, the cellulosic biomass is subjected to conditions such as 190-210 °C and a pH of 1.5-2.5 in the second hydrolysis unit (18b), to obtain a cellulose-derived slurry that is transferred to the container (1) via an inlet of the container (14). Typical retention times are 5-10 minutes per step. Before the slurry enters the container (1), it is neutralized by addition of a base, such as NaOH or ammonia. The container (1) is connected to a separator (2), and a part of the cellulose-derived slurry is transferred via a third transferring arrangement (6), such as a pipe, to the separator (2) via a slurry inlet (2a) of the separator. The separator may for example be a band filter. The transferring of the slurry to the separator (2) is aided by a third pump (10), arranged on the third transferring arrangement (6). The separator (2) is provided with at least one filter for separating a liquid fraction from the slurry. The remaining suspended solids fraction is returned to the container (1) via the suspended solids fraction outlet (2c) of the separator and a fourth transferring arrangement (7), while the liquid fraction is transferred via a liquid fraction outlet (2b) of the separator (2), through a first transferring arrangement (4), e.g. a pipe, to a fermentor (3) via a fermentor inlet (3a) of the fermentor. The transferring of the liquid fraction through the first transferring arrangement (4) is aided by a first pump (8), arranged on the first transferring arrangement (4). The fermentor (3) comprises a fermenting organism, such as flocculating *Saccharomyces cerevisiae,* that ferments the fermentable saccharides in the liquid fraction to the target chemical, e.g. ethanol. The temperature of the fermentor (3) is regulated e.g. with a cooler (13), to a temperature of around 35 °C, which normally is close to the optimal temperature of the fermenting reaction when a yeast is employed. The obtained fermented liquid, which thus comprises the target chemical, is withdrawn from the fermentor outlet (3b) arranged at the top of the fermentor (3), at a speed that allows the fermenting organism to sediment in the fermentor (3). Consequently, the fermenting organism is to a large extent retained in the fermentor (3). The fermented liquid is returned to the container (1) from the fermentor outlet (3b) through a second transferring arrangement (5), e.g. a pipe. The transferring of the fermented liquid through the second transferring arrangement (5) is aided by a second pump (9), arranged on the second transferring arrangement (5). The processes of transferring a part of the cellulose-derived slurry from the container (1) to the separator (2), separating a liquid fraction and transferring the liquid fraction to the fermentor (3) while returning a suspended solids fraction to the container (1), and returning the fermented liquid to the container (1) is continuously repeated. Since the fermented liquid, comprising the target chemical, is returned to the container (1), the concentration of the target chemical is gradually increased in the system. The above-mentioned processes are continuously repeated until a desired concentration of the target chemical is achieved in the container (1). Then, the whole content of the container (1) is transferred to a target recovery unit (16), which may comprise a distillation column. The content of the container (1) may pass at least one filter (17) to decrease the suspended solids content while being transferred to the target recovery unit (16). The target chemical, e.g. ethanol, is then recovered in the target recovery unit (16), e.g. by means of distillation in a distillation column. Further, the system disclosed above could also be equipped with a heat exchanger that facilitates exchange of heat between the first transferring arrangement (4) and the second transferring arrangement (5) to further optimize the energy usage in the system.

### Example 2. Method and system for providing a target chemical, in which the cellulose-derived slurry is obtained by enzymatic hydrolysis of cellulosic biomass.

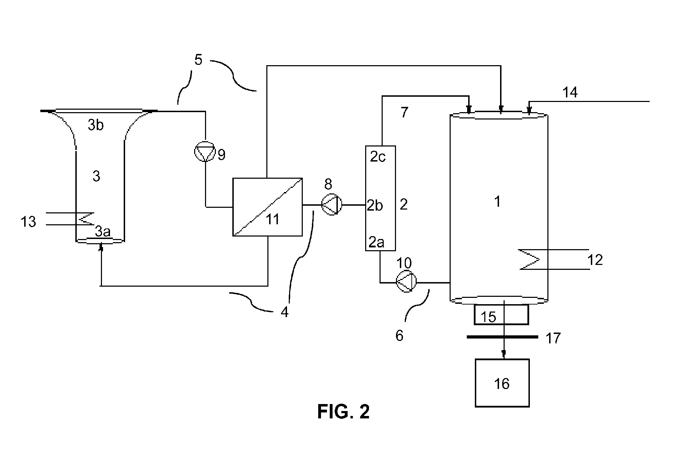

Figure 2 illustrates an embodiment of the disclosed method and system, in which the cellulose-derived slurry is obtained by enzymatic hydrolysis. The system for providing the target chemical comprises a container (1) for containing a cellulose-derived slurry. The cellulose-derived slurry, having a suspended solids content of 10-15 %, preferably 10-13 %, is obtained by providing pretreated cellulosic biomass to the container (1) via an inlet of the container (14). The cellulosic biomass may for example have been pretreated by dilute acid treatment and/or steam explosion. The pretreated cellulosic biomass is then subjected to enzymatic hydrolysis in the container (1) using saccharification enzymes. These enzymes catalyze the hydrolysis of polysaccharides in the pretreated cellulosic biomass to fermentable saccharides, such as disaccharides and monosaccharides. Gradual digestion of the pretreated cellulosic biomass may lead to a lower suspended solids content in the container than in the ingoing feed of pretreated cellulosic biomass. The catalytic turnover rate of saccharification enzymes used in the container (1) may be highest at a temperature around 65 °C, and therefore a heater (12) is arranged on the container (1) to heat the cellulose-derived slurry in the container (1). The container (1) is connected to a separator (2). A part of the cellulose-derived slurry is transferred via a third transferring arrangement (6), such as a pipe, to the separator (2) via a slurry inlet (2a) of the separator. The transferring of the slurry to the separator (2) is aided by a third pump (10), arranged on the third transferring arrangement (6). The separator (2) is provided with at least one filter for separating a liquid fraction from the slurry. The remaining suspended solids fraction is returned to the container (1) via the suspended solids fraction outlet (2c) of the separator and through a fourth transferring arrangement (7), while the liquid fraction is transferred via a liquid fraction outlet (2b) of the separator (2), through a first transferring arrangement (4), e.g. a pipe, to a fermentor (3) via a fermentor inlet (3a) of the fermentor. The transferring of the liquid fraction through the first transferring arrangement (4) is aided by a first pump (8), arranged on the first transferring arrangement (4). The fermentor (3) comprises a fermenting organism, such as flocculating *Saccharomyces cerevisiae,* that ferments the fermentable saccharides in the liquid fraction to the target chemical, e.g. ethanol. The obtained fermented liquid, which thus comprises the target chemical, is withdrawn from the fermentor outlet (3b), arranged at the top of the fermentor (3), at a speed that allows the fermenting organism to sediment in the fermentor (3). Consequently, substantially all of the fermenting organism is retained in the fermentor (3). The fermentation rate of the fermenting organism has a temperature maximum of about 35 °C, i.e. lower than the temperature of the slurry in the container (1). Therefore, a cooler (13)

is arranged on the fermentor (3) to decrease the temperature in the fermentor (3) to a desired level. The fermented liquid is returned to the container (1) from the fermentor outlet (3b) through a second transferring arrangement (5), e.g. a pipe. The transferring of the fermented liquid through the second transferring arrangement (5) is aided by a second pump (9), arranged on the second transferring arrangement (5). Further, the first transferring arrangement (4) and the second transferring arrangement (5) are in thermal contact in a heat exchanger (11), which means that the temperature of the liquid fraction that is transferred in the first transferring arrangement (4) is decreased and the temperature of the fermented liquid that is transferred in the second transferring arrangement (5) is increased, thus facilitating an efficient energy conservation in the system and less use of the heater (12) arranged on the container (1) and the cooler (13) arranged on the fermentor (3). The processes of transferring a part of the cellulose-derived slurry from the container (1) to the separator (3), separating a liquid fraction and transferring the liquid fraction to the fermentor (3) while returning a suspended solids fraction to the container (1), and returning the fermented liquid to the container (1) is continuously repeated. Since the fermented liquid, comprising the target chemical, is returned to the container (1), the concentration of the target chemical is gradually increased in the system. The above-mentioned processes are continuously repeated until a desired concentration of the target chemical is achieved in the container (1). Then, the whole content of the container (1) is transferred to a target recovery unit (16), which may comprise a distillation column. The whole content of the container (1) may pass an enzyme recovery arrangement (15), which comprises affinity means for the saccharification enzymes so the enzymes can be recovered and reused in further hydrolysis reactions in the container (1). Further, the content of the container (1) may pass at least one filter (17) to decrease the suspended solids content while being transferred to the target recovery unit (16). The target chemical, e.g. ethanol, is then recovered in the target recovery unit (16), e.g. by means of distillation in a distillation column.

### Example 3. System for withdrawing a liquid comprising ethanol from a fermentor while retaining a flocculating yeast inside the fermentor

Fig. 6 shows an example of a system for withdrawing liquid comprising ethanol from a fermentor while a flocculating yeast is retained inside the fermentor. The fermentor (41) is connected to an inlet tube (42) and an outlet tube (44). In the illustrated example, four pipes (43) are connected to the outlet tube and extend below the surface (47) of the liquid in the fermentor. Flocculating yeast (46) is present in the liquid of the fermentor and an agitator (45) is provided to homogenize the liquid-yeast mixture (and thereby provide for an efficient fermentation). Liquid comprising ethanol produced by the flocculating yeast (46) is withdrawn from the pipes (43) by applying an underpressure to the outlet tube (44). The dimensions, such as the diameters, of the pipes (43) are configured so that the rate at which the liquid comprising the ethanol rises in the pipes (43) is lower than the average settling rate of the flocculating yeast (46). Further, the arrangement of the agitator and the level of agitation are such that turbulent motions of the liquid at the openings of the outlet tubes (43) extending below the surface (47) of the liquid in the fermentor are minimized. Thus, liquid comprising the ethanol is withdrawn from the fermentor (41) while the flocculating yeast (46) is retained inside the fermentor (41).

### Experimental embodiments

The following non-limiting examples will further illustrate the present invention.

### Example 4. Ethanol preparation comprising enzymatic hydrolysis

### Materials and methods

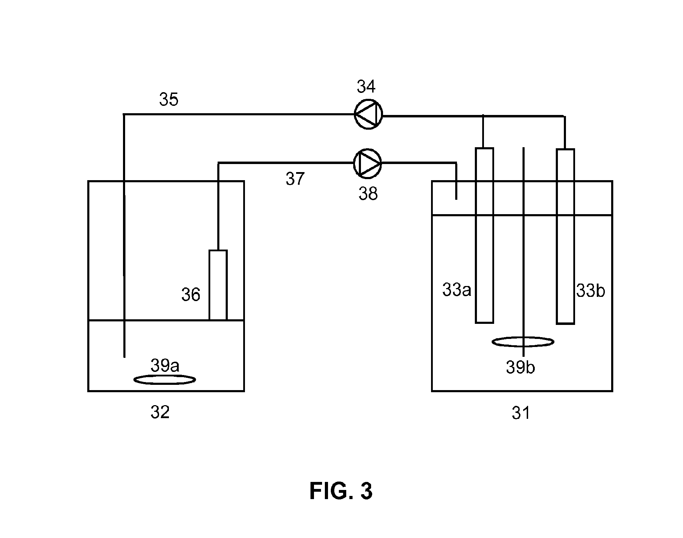

A schematic overview of the experimental set-up of Example 4 is illustrated in Fig. 3. A hydrolysis vessel (31) was filled with 2500 ml cellulose-derived slurry, initially having a suspended solids content of 17.2 % SS (w/w). The temperature of the hydrolysis vessel (31) was 40 °C and the pH was around 5.0. Saccharification enzymes (Celluclast from Novozymes) was added to the hydrolysis vessel (31) in an amount of 7.5 FPU/g of dry material. Further, an excess of beta-glucosidase was also added to the hydrolysis vessel (31). The enzymatic hydrolysis reaction in the hydrolysis vessel (31) was performed in an anaerobic environment during continuous stirring by using a first stirrer (39b). A liquid fraction was separated from the hydrolysis vessel (31) by means of two filters (33a and 33b) and was transferred in a first tubing (35) using a first pump (34) to a fermentation vessel (32) of 300 ml . A flocculating yeast in an amount of about 9 g/l was initially added to the fermentation vessel (32). The temperature of the fermentation vessel (32) was 35 °C and the pH was around 5.0. The fermentation in the fermentation vessel (32) was performed in an anaerobic environment during continuous stirring by the use of a second stirrer (39a). A fermentation liquid was slowly withdrawn through an "internal settler" (36), a cylinder through which the flocculating yeast cells could not pass since their sedimentation velocity was higher than the movement upwards of the liquid. A fermentation liquid was transferred back to the hydrolysis vessel (31) in a second tubing (37) using a second pump (38) while the yeast was retained in the fermentation vessel (32).

### Results

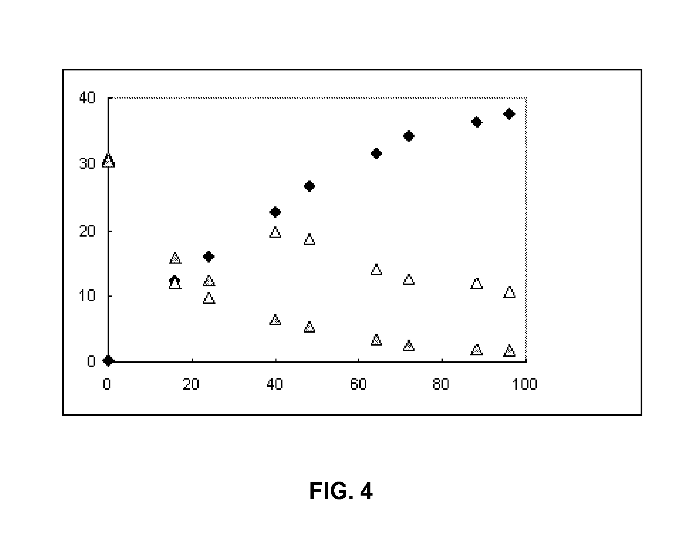

The experiment was conducted during 96 hours. The average recirculation flow was measured to about 200 ml/hour, i.e. the whole volume of the liquid content hydrolysis vessel (31) was recirculated in 12.5 h. The ethanol, glucose and mannose concentrations in the hydrolysis vessel were measured throughout the experiment, and the results are displayed in Fig. 4. After 96 h, the ethanol concentration in the hydrolysis vessel was about 37.6 g/l. A slightly higher amount was measured in the fermentation vessel. This means that a concentration of fermentable saccharides of about 12.3 g/l was left in the system after 96 h. In theory, if the experiment was conducted for a longer period of time, this amount could lead to an additional 4-5 g/l ethanol, i.e. result in a total ethanol concentration in the hydrolysis vessel of over 40 g/l.

The glucan turnover was estimated to about 70 %. The ethanol yield was measured to about 73% of the highest theoretical level, which probably means that ethanol was evaporating in the system during the experiment and that a higher concentration of ethanol was produced than what could be measured.

Hence, Example 4 is a proof-of-concept of the method and system of the present disclosure and shows that a high concentration of ethanol can be produced.

### Example 5. Ethanol preparation without enzymatic hydrolysis

### Materials and methods

The same experimental set-up as described in Example 4 was used, but no enzymes were added to the hydrolysis vessel (31). Further, glucose and mannose were added to the hydrolysis vessel (31) to an initial concentration of 11 g/l and 16 g/l, respectively, and the temperature of the hydrolysis vessel (31) was 50 °C.

### Results

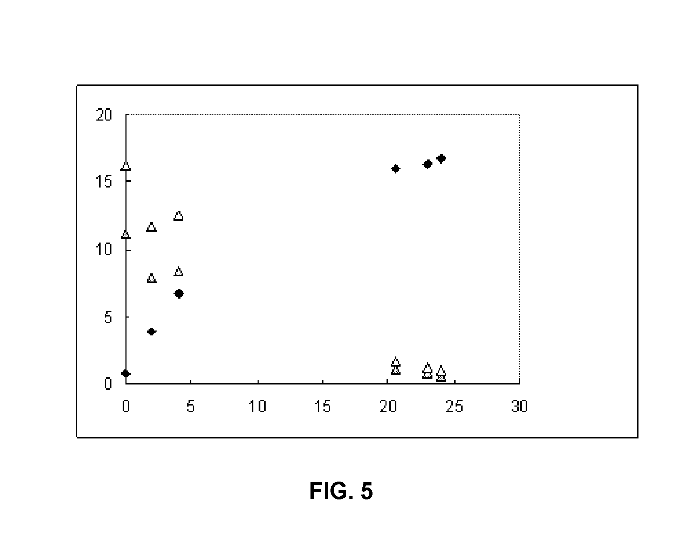

The experiment was conducted during 25 hours. The ethanol, glucose and mannose concentrations in the hydrolysis vessel was measured throughout the experiment, and the results are displayed in Fig. 5. The sugar concentrations decreased more rapidly compared to Example 4 and all the sugars were more or less consumed after 25 hours. This was probably because no further saccharides could be created due to lack of saccharification enzymes in the hydrolysis vessel. The ethanol concentration after 25 hours was about 16 g/l, which was lower compared to the concentration obtained in Example 4. However, this may also be explained by a lower initial saccharide concentration and the lack of saccharification enzymes during the course of the experiment. The ethanol yield was 86 % of the maximum theoretical ethanol yield.

Consequently, the results obtained in Example 5 is also a good proof-of-concept of the disclosed method and system for preparing a target chemical.

## Claims

1. A method for providing a target chemical, comprising:
a) providing a cellulose-derived slurry comprising fermentable saccharides in a container;
b) separating a liquid fraction comprising part of said fermentable saccharides from said cellulose-derived slurry;
c) transferring said liquid fraction to a fermentor;
d) fermenting fermentable saccharides in said liquid fraction in said fermentor to provide a fermented liquid comprising said target chemical; and
e) returning said fermented liquid to said container.

2. The method according to claim 1, wherein the separating of step b) is performed in a separator providing said liquid fraction and a suspended solids fraction, which is returned to said container.

3. The method according to any previous claim, wherein the target chemical is recovered from the fermented liquid during step e).

4. The method according to any one of claims 1-3, further comprising:
f) recovering the target chemical from at least part of the content of said container in a target recovery unit.

5. The method according to any previous claim, wherein step d) comprises contacting said liquid fraction with a fermenting organism to obtain said fermentation liquid.

6. The method according to claim 5, wherein said fermenting organism is retained in said fermentor.

7. The method according to any previous claim, wherein said cellulose-derived slurry of step a) is obtained by a method comprising:
a1) providing optionally pretreated cellulosic biomass; and
b1) subjecting said cellulosic biomass from step a1) to hydrolysis
in at least one hydrolysis unit separate from said container to obtain said cellulose-derived slurry.

8. The method according to any one of claims 1-6, wherein said cellulose-derived slurry of step a) is obtained by a method comprising the steps of:
a2) providing optionally pretreated cellulosic biomass; and
b2) subjecting said cellulosic biomass to enzymatic hydrolysis in
said container to obtain said cellulose-derived slurry.

9. The method according to any one of claims 1-8, wherein the temperature of said slurry in said container is between 30 and 100 °C, such as between 40 and 70 °C, and the temperature of said liquid fraction in said fermentor is between 25 and 90 °C, such as between 28 and 38 °C.

10. The method according to any one of claims 7-9, wherein said cellulosic biomass is lignocellulosic biomass, such as sugarcane bagass or wood.

11. The method according to any previous claim, wherein step e)
comprises heating said fermentation liquid by bringing it into thermal contact with said liquid fraction of step c).

12. Use of a fermented, cellulose-derived hydrolysate for extracting fermentable saccharides from a cellulose-derived slurry, wherein said cellulose-derived slurry has a suspended solids content of from 5 % to about 30 %.

13. A system for fermenting sugars to a target chemical, comprising:
a container for containing a cellulose-derived slurry comprising fermentable saccharides;
a separator for separating a liquid fraction comprising fermentable saccharides from said slurry, which separator is connected to said container;
a fermentor for fermenting at least part of said fermentable saccharides in said liquid fraction to yield a fermented liquid, which fermentor comprises an inlet and an outlet;
a first connection connecting said separator and said fermentor inlet
and
a second connection connecting said fermentor outlet and said container.

14. The system according to claim 13, wherein said container is adapted
for recovery of said target chemical from said container.

15. The system according to any one of claims 13-14, further comprising
at least one hydrolysis unit for hydrolyzing cellulosic biomass and providing said cellulose-derived slurry, which at least one hydrolysis unit is connected to said container.
